# EUROPEAN PATENT SPECIFICATION

(11) **EP 2 411 059 B1**
(45) Date of publication and mention of the grant of the patent: **21.01.2015**
(21) Application number: 10710415.0
(22) Date of filing: 19.03.2010
(51) Int. Cl.: A61L 9/12

(54) **DISPENSER FOR AN AIR FRESHENER**
SPENDER FÜR EINEN LUFTERFRISCHER
DISTRIBUTEUR DE DÉSODORISANT

(30) Priority: 28.03.2009 GB 0905411
(43) Date of publication of application: 01.02.2012
(73) Proprietor: 3sixty Link Design To Supply Limited, Exeter, Devon EX2 7HU (GB)
(72) Inventor: SMITH, Nigel Peter, Devon TQ12 6HH (GB)
(74) Representative: Ratcliffe, Susan Margaret
(86) International application number: PCT/GB2010/050465
(87) International publication number: WO 2010/112895

(56) References cited:
- WO-A1-98/25651
- WO-A1-99/03514
- WO-A1-2008/037101
- GB-A- 2 181 649

## Description

### Field of the Invention

This invention relates to a dispenser and, in particular, to a device or element of a device for dispensing an air modifying agent into the surrounding atmosphere.

### Background to the Invention

Air modifying agents, typically comprising air fresheners, de-odourisers, insect repellants and the like, can be dispensed in many different ways. One way is to entrain the agent in a gel such that the agent evaporates when exposed to the atmosphere. The gel may be packed in a sealed container, which container is opened to the surrounding atmosphere, at point of use, to allow volatile active materials entrained in the gel to disperse.

Examples of alternatives to entraining the active ingredient or agent in gel comprise incorporating the agent in a liquid, the liquid being in contact with mat or wick, or a combination of the two. In use, the agent evaporates from the mat or wick. Examples of this type of device are described in GB Patent Application 2 181 649 which discuses a simple wick and pad arrangement and in Published International Patent Application Nos WO 98/25651 which discusses a pad type vapor emanating surface covered by a peelable seal, WO 2008/037101 (a passive wick and pad type diffuser) and WO 99/03514 (capillary acting air freshener device).

Liquid-based air fresheners of the mat or wick type present packaging and/or transport issues. The units, or refill units, need to be securely packed to avoid leakage while passing through the distribution chain, yet be easily opened and placed in a operating position, without causing spillage, when the unit is to be used.

Further, the unit must be capable of inexpensive manufacture yet, when opened for use, must dispense the air modifying agent in an efficacious manner.

European Patent No. 1 529 004 describes a fuel tin in which a fuel is sealed within a simple container by means of a sealing foil. The sealing foil has lines of weakening therein whereby the foil may be broken to create an opening which exposes the fuel within the container. Embodiments are described which include pull tabs to facilitate breaking of the foil about the lines of weakening.

Whilst European Patent No. 1 529 004 describes a container of active material which can be easily opened, the surface of the material is directly exposed upon opening and there is a substantial risk that the jerking action, which is inevitable upon opening, will lead to some spillage of the material within the container. Likewise, if the container is tipped on to its side, there is nothing to prevent fuel therein from spilling directly through the opening created in the lid.

It is an object of this invention to provide a form of dispenser for an air modifying agent which goes at least some way in addressing the aforementioned needs or drawbacks; or which will at least provide a novel and useful choice.

### Summary of the Invention

Accordingly the invention provides a dispenser for a liquid modifying agent said dispenser including:
a container body;
a closing lid engaged with said container body said closing lid having a displaceable or removable section;
a wicking member within said container;
a liquid absorbent emanation surface within said container and in contact with said wicking member,
said dispenser being characterized in that said closing lid biases said emanation surface into contact with said wicking member;
and with said displaceable or removable section intact, said closing lid maintains said emanation surface under compression such that removal of said displaceable or removable section allows expansion of said emanation surface; with there being at least one locating member which projects into the container body to define a space capable of receiving the wicking member with said at least one locating member also loosely supporting the wicking member so said wicking member is positively located within the container body.

Preferably said closing lid has a line of weakening therein which defines said displaceable or removable section.

Preferably a pull-tab is attached to said displaceable or removable section.

Preferably said locating means comprises a plurality of webs projecting inwardly from said container body to define a space capable of receiving and supporting said wicking member.

Preferably said locating means also serves to locate said emanation surface relative to said closing lid.

Preferably said locating means is configured to allow said emanation surface to be displaced, at least in part, during opening.

### Brief Description of the Drawings

One form of dispenser embodying the various aspects of the invention will now be described with reference to the accompanying drawing in which:
Figure 1 shows an isometric view of a dispenser according to the invention;
Figure 2 shows a vertical cross-section through the dispenser shown in Figure 1; and
Figure 3 shows a view along the line X-X in Figure 2.

### Detailed Description of Working Embodiment

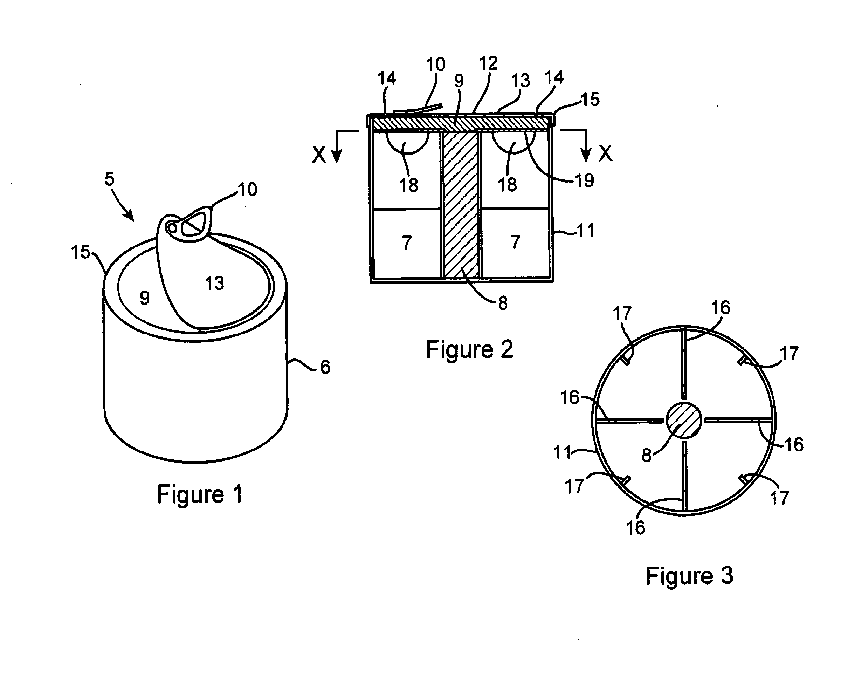

Referring to Figure 1, the invention provides a dispenser 5 for dispensing an air modifying agent. In the form shown the dispenser includes a sealed container 6 in which is contained a liquid air-modifying agent 7 (Figure 2). Prior to use, the container is breached, in a manner described below, so that the air-modifying agent can disperse into the surrounding atmosphere.

Typically the air modifying agent will be a fragrance but it could also comprise, or include, insect repellants, anti-oxidising agents, de-odourising agents etc. The precise make-up of the air-modifying agent does not form part of the invention.

The dispenser as described herein is of the liquid wick type and, to that end, includes a wicking member 8 extending down into the liquid 7, and an emanation surface 9 which contacts the wicking member 8, draws the liquid 7 there-from, and provides a surface from which, in use when the container is opened, the liquid 7 can evaporate or otherwise disperse into the surrounding atmosphere.

The container 6 includes a closing lid to seal the container whilst passing through the distribution chain. Defined within the closing lid is a displaceable or removable section which, when displaced or removed, exposes the emanation surface to the surrounding atmosphere. Unlike the fiddly screw caps or fragile metal foil closures of prior art devices of this type, the invention provides a robust yet easily removed section which preferably incorporates a ring-pull tab 10 to facilitate opening.

In the form shown the container 6 is defined by a container body 11 and a closing lid 12, the lid 12 being crimped or otherwise sealed to the upper edge of the container body 11. The lid 12, in turn, has a removable section 13 defined by a line of weakening 14. Upon manipulation of the ring-pull tab 10, which is attached to the closure13, the lid can be ruptured along the line 14 and the section 13 removed and discarded.

As can best be seen in Figure 2, the emanation surface 9 is conveniently provided by an absorbent pad which may be, for example, a cellulose pad of, say, 3-6mm in thickness. The precise form of the pad is not an essential feature of the invention and the composition, thickness and density of the pad may be varied according to the type of liquid being dispensed and the evaporation performance required. Those skilled in the art will appreciate that pads of this type have a degree of flexibility and compressibility.

The pad 9 contacts the upper end of the wicking member 8 and is preferably compressed against the wicking member 8 by the lid 12, the rim 15 of the lid remaining, after removal of the section 13, for this very purpose. Compression of the pad 9 against the wicking member 8 markedly increases the transfer of liquid to the pad 9 and thus enhances the output of the dispenser.

The wicking member 8 may be of any known form and can also be formed from a cellulose and/or polyester material. As with the pad 9, the precise nature of the wicking member is not essential to the invention.

Another distinguishing aspect of the invention is that the wicking member 8 is positively located within the container 6. In the form shown, at least one and, as shown four, locating members such as webs 16 project inwardly to define a space capable of receiving and loosely supporting the wicking member 8 yet, at the same time, not restricting the liquid 7 to any particular part of the container. The webs 16 are preferably formed integrally with the container body 11 and preferably further serve to locate and support the pad 9 and to hold the pad in compression against the lower surface of the closing lid 12. To prevent the pad sagging between the webs 16 further, intermediate supports 17 may also be provided.

The upper edges of the webs 16 are preferably provided with downwardly extending recesses 18. These provide clearance when the pad 9 is deflected downwardly due to the section 13 being displaced at least partly into the container as the ring-pull tab 10 is raised to rupture the section 13 from the remainder of the lid 12.

When the lid is intact, the pad is maintained under compression against the webs 16. If, during opening, the active liquid is splashed into contact with the pad 9, the expansion of the pad will accommodate that liquid and thus prevents spillage. We have found that a container of the type described can even be displaced on to its side after opening, and no external spillage will occur for a significant time. The chances of spillage can be further minimized by positioning a liquid impermeable membrane 19 beneath the pad 9, the membrane 19 having a central aperture to allow the wicking member 8 to contact the pad 9. The membrane 19 is flexible and/or elastic to allow downward deflection of the pad during operation of the pull-tab 10.

Alternatively the membrane 19 could be replaced by a more rigid annular member having an annular groove to engage in the recesses 18 and thus provide clearance for deflection of the pad during operation of the pull-tab 10.

It will thus be appreciated that the various aspects of the invention provide a dispenser for an air modifying agent which, at least in the case of the embodiment described above, can be easily and inexpensively formed whilst providing a highly efficient dispensing action and minimizing the risk of spillage. A dispenser as described can be configured as a stand-alone unit or can be configured for operation with an air moving device such as a fan.

## Claims

1. A dispenser (5) for a liquid modifying agent (7), said dispenser including:
a container body (11);
a closing lid (12) engaged with said container body (11), said closing lid having a displaceable or removable section (13);
a wicking member (8) within said container (6);
a liquid absorbent emanation surface (9) within said container and in contact with said wicking member (8),
with there being at least one locating member (16) which projects into the container body (11) to define a space capable of receiving the wicking member with said at least one locating member also loosely supporting the wicking member (8) so said wicking member is positively located within the container body (11),
said dispenser (5) being **characterized in that**:
said closing lid (12) biases said emanation surface (9) into contact with said wicking member (8);
and with said displaceable or removable section (13) intact, said closing lid (12) maintains said emanation surface (9) under compression such that removal of said displaceable or removable section (13) allows expansion of said emanation surface.

2. A dispenser as claimed in claim 1 wherein said closing lid (12) has a line of weakening (14) therein which defines said displaceable or removable section (13).

3. A dispenser as claimed in claim 1 or claim 2 wherein a pull-tab (10) is attached to said displaceable or removable section (13).

4. A dispenser as claimed in any one of claims 1 to 3 wherein said locating means comprises a plurality of webs projecting inwardly from said container body to define a space capable of receiving and supporting said wicking member.

5. A dispenser as claimed in any preceding claim wherein said locating means (16) also serves to locate said emanation surface (9) relative to said closing lid (12).

6. A dispenser as claimed in any preceding claim wherein said locating means (16) is configured (18) to allow said emanation surface to be displaced, at least in part, during displacement or removal of said displaceable or removable section (13).

## Patentansprüche

1. Spender (5) für ein flüssiges modifizierendes Mittel (7), wobei der Spender Folgendes umfasst:
einen Behälterkörper (11);
einen Verschlussdeckel (12), der mit dem Behälterkörper (11) in Eingriff steht, wobei der Verschlussdeckel einen verschiebbaren oder entfernbaren Abschnitt (13) aufweist;
ein Dochtelement (8) in dem Behälter (6);
eine Flüssigkeit aufsaugende Ausstrahlungsfläche (9) in dem Behälter, die mit dem Dochtelement (8) in Kontakt steht,
wobei zumindest ein Lokalisationselement (16) vorhanden ist, das in den Behälterkörper (11) vorsteht, um einen Raum zu definieren, der das Dochtelement aufnehmen kann, wobei das zumindest eine Lokalisationselement das Dochtelement (8) auch lose stützt, so dass das Dochtelement positiv in dem Behälterkörper (11) angeordnet ist,
wobei der Spender (5) **dadurch gekennzeichnet ist, dass**:
der Verschlussdeckel (12) die Ausstrahlungsfläche (9) in Kontakt mit dem Dochtelement (8) vorspannt;
und, wenn der verschiebbare oder entfernbare Abschnitt (13) intakt ist, der Verschlussdeckel (12) die Ausstrahlungsfläche (9) unter Kompression hält, so dass die Entfernung des verschiebbaren oder entfernbaren Abschnitts (13) eine Ausweitung der Ausstrahlungsfläche gestattet.

2. Spender nach Anspruch 1, worin der Verschlussdeckel (12) eine Schwächungslinie (14) darin aufweist, die den verschiebbaren oder entfernbaren Abschnitt (13) definiert.

3. Spender nach Anspruch 1 oder Anspruch 2, worin eine Zuglasche (10) an dem verschiebbaren oder entfernbaren Abschnitt (13) befestigt ist.

4. Spender nach einem der Ansprüche 1 bis 3, worin das Lokalisationsmittel eine Vielzahl von Bahnen umfasst, die von dem Behälterkörper nach innen vorstehen, um einen Raum zu definieren, der das Dochtelement aufnehmen und stützen kann.

5. Spender nach einem der vorhergehenden Ansprüche, worin das Lokalisationsmittel (16) auch dazu dient, die Ausstrahlungsfläche (9) im Verhältnis zu dem Verschlussdeckel (12) örtlich festzulegen.

6. Spender nach einem der vorhergehenden Ansprüche, worin das Lokalisationsmittel (16) ausgelegt (18) ist, eine zumindest teilweise Verschiebung der Ausstrahlungsfläche während der Verschiebung oder Entfernung des verschiebbaren oder entfernbaren Abschnitts (13) zu gestatten.

## Revendications

1. Distributeur (5) pour un agent de modification liquide (7), ledit distributeur comprenant:
un corps de récipient (11);
un couvercle de fermeture (12) qui est engagé avec ledit corps de récipient (11), ledit couvercle de fermeture présentant une section déplaçable ou amovible (13) ;
un élément de mèche (8) à l'intérieur dudit récipient (6);
une surface d'émanation d'absorption de liquide (9) à l'intérieur dudit récipient et en contact avec ledit élément de mèche (8),
au moins un élément de positionnement (16) qui fait saillie dans le corps de récipient (11) afin de définir un espace capable de recevoir l'élément de mèche avec ledit au moins un élément de positionnement qui supporte également librement l'élément de mèche (8) de telle sorte que ledit élément de mèche soit situé positivement à l'intérieur du corps de récipient (11),
ledit distributeur (5) étant **caractérisé en ce que**:
ledit couvercle de fermeture (12) pousse ladite surface d'émanation (9) en contact avec ledit élément de mèche (8); et
avec ladite section déplaçable ou amovible (13) intacte, ledit couvercle de fermeture (12) maintient ladite surface d'émanation (9) sous compression de telle sorte que l'enlèvement de ladite section déplaçable ou amovible (13) permette l'expansion de ladite surface d'émanation.

2. Distributeur selon la revendication 1, dans lequel ledit couvercle de fermeture (12) présente une ligne d'affaiblissement (14) qui définit ladite section déplaçable ou amovible (13).

3. Distributeur selon la revendication 1 ou la revendication 2, dans lequel un onglet à tirer (10) est attaché à ladite section déplaçable ou amovible (13).

4. Distributeur selon l'une quelconque des revendications 1 à 3, dans lequel lesdits moyens de positionnement comprennent une pluralité de bandes qui font saillie intérieurement à partir dudit corps de récipient afin de définir un espace capable de recevoir et de supporter ledit élément de mèche.

5. Distributeur selon l'une quelconque des revendications précédentes, dans lequel lesdits moyens de positionnement (16) servent également à positionner ladite surface d'émanation (9) par rapport audit couvercle de fermeture (12).

6. Distributeur selon l'une quelconque des revendications précédentes, dans lequel lesdits moyens de positionnement (16) sont configurés (18) de manière à permettre à ladite surface d'émanation d'être déplacée, au moins en partie, pendant le déplacement ou l'enlèvement de ladite section déplaçable ou amovible (13).
